# EUROPEAN PATENT SPECIFICATION

(11) **EP 1 900 343 B1**
(45) Date of publication and mention of the grant of the patent: **21.10.2015**
(21) Application number: 07008364.7
(22) Date of filing: 31.01.2001
(51) Int. Cl.: A61F 2/24

(54) **Stent valves**
Stentklappen
Valvules d'endoprothèse vasculaire

(30) Priority: 31.01.2000 US 179195 P
(43) Date of publication of application: 19.03.2008
(62) Divisional of application: 01905265.3
(73) Proprietor: Cook Biotech Incorporated, West Lafayette, IN 47906 (US)
(72) Inventor: Obermiller, Joseph F., West Lafayette, IN 47906 (US); Osse, Francisco, Jose, CEP-05428001, San Paulo, SP/BR (BR); Thorpe, Patricia, E., Iowa City, IA 52240 (US)
(74) Representative: Atkinson, Peter Birch

(56) References cited:
- EP-A- 0 808 614
- EP-A- 0 850 607
- WO-A-99/15224
- US-A- 5 855 601

## Description

### Background

### Technical field of the invention:

The invention includes a medical device and more specifically to a valve found generally within a frame. In preferred devices the frame is comprised of a radially expandable stent which can be delivered through a delivery device such as a catheter.

### Background of the invention:

Lower extremity venous hypertension in addition to venous insufficiency is a major cause of morbidity in the United States. Symptoms of venous disease include lower extremity edema, varicosities, skin pigmentation changes, skin ulceration, and general poor circulation. One solution to this problem is to replace the defective valve or the vein with a valve assembly.

Current valves include a pressure responsive, pressure directed ball movement valve assemblies. The problem with mechanical ball valves is that mechanical valves are susceptible to clot formation. Additionally, there are problems associated with long-term wear and tear on the device.

Artificial valves such as biological valves are also known. Biological valves include homografts, allografts, and xenografts. Problems associated with some biological valves include the supply of the valves, immunity response, or problems associated with matching the size with the donor.

Finally other problems associated with valve repair include placement problems in which the device cannot be repositioned once it is ejected from the placement catheter, leakage that occurs around the valve, and emboli formation.

US 5,855,597 discloses a stent valve device for use in repairing a damaged cardiac valve. The stent comprises two to eight open-centred, star-shaped members with outwardly directed points that are bent so as to face away from a plane defined by the inwardly directed points. The star shaped members are interconnected to form a "chain" to form a radially expandable stent. A tri-cuspid valve, made of any suitable flexible and bio-compatible material, is located in the central opening of the stent. A catheter delivery system is used to deliver the stent with the tri-cuspid valve to the desired site.

In light of this background, there remains a need for alternative and improved devices and methods for providing valvular function within vessels of the body. The present invention is addressed to these needs.

### Summary of the Invention

The invention provides a stent valve device for delivery by catheter to a vessel site (80) in the body, comprising:
a radially expandable cylindrical stent, the radially expandable cylindrical stent having a collapsed configuration for delivery and an expanded configuration for deployment at the vessel site, the radially expandable cylindrical stent (20) having a proximal stent end, a distal stent end, a stent lumen, and a stent diameter and a stent perimeter,
a valve located within the lumen of the radially expandable cylindrical stent, the valve comprising biocompatible material and at least two leaflets, the valve having a valve orifice between the at least two leaflets, the valve orifice extending substantially across the stent diameter, wherein
   (a) the valve has a proximal portion connected to a proximal portion of the radially expandable cylindrical stent and a distal portion connected to a distal portion of the radially expandable cylindrical stent;
   (b) at the locations between adjacent leaflets, the valve orifice terminates 1 to 5 millimeters before reaching the perimeter of the radially expandable cylindrical stent;
   (c) the valve material is enclosed within the radially expandable cylindrical stent;
      characterized in that
   (d) reinforcement mechanisms are located between the orifice perimeter and the stent perimeter at the locations between adjacent leaflets at valve-stent-opening connection points; thereby reinforcing said connection points against wear and tear due to opening and closing of the valve and permitting increased structural integrity of the stent valve device.

The frame is comprised of a radially-expandable stent (including especially a self-expanding stent), which can be delivered through a delivery device such as a catheter, and then deployed and expanded at a target site in a body lumen such as an artery or vein. For example, in one preferred use, such a stent and method are used to treat incompetent veins in the legs or feet.

### Brief Description of the Drawings

FIGs. 1A to 3 demonstrate a stent.
FIGs. 4 to 8 demonstrate a valve.
FIGs. 9 to 11 demonstrate exemplary ways of attaching a plurality of stents.
FIGs. 12 to 15 demonstrate exemplary embodiments of the valve configuration in a variety of stent embodiments.
FIG. 16 demonstrates one aspect of the invention in situ.
FIGs. 17 to 19 demonstrate other alternative embodiments.
FIG. 20 depicts a medical including a stent valve and a delivery device for the stent valve.

### Detailed description of the invention

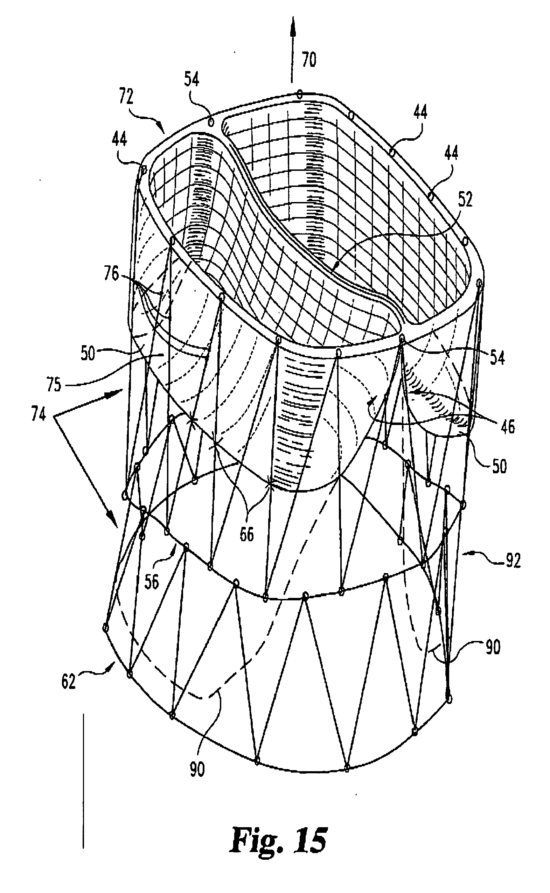

With reference to FIG. 15, shown is one embodiment of the present invention. The invention includes a frame such as a wire stent that has a lumen extending therethrough. Near one end of the stent is the valve assembly comprising some leaflets or cusps. A valve opening is generally located between the leaflets through which fluid flows. Although shown as a two leaflet valve, equally the invention can comprise, in any embodiment described herein, at least two leaflets such as two, three or four leaflets.

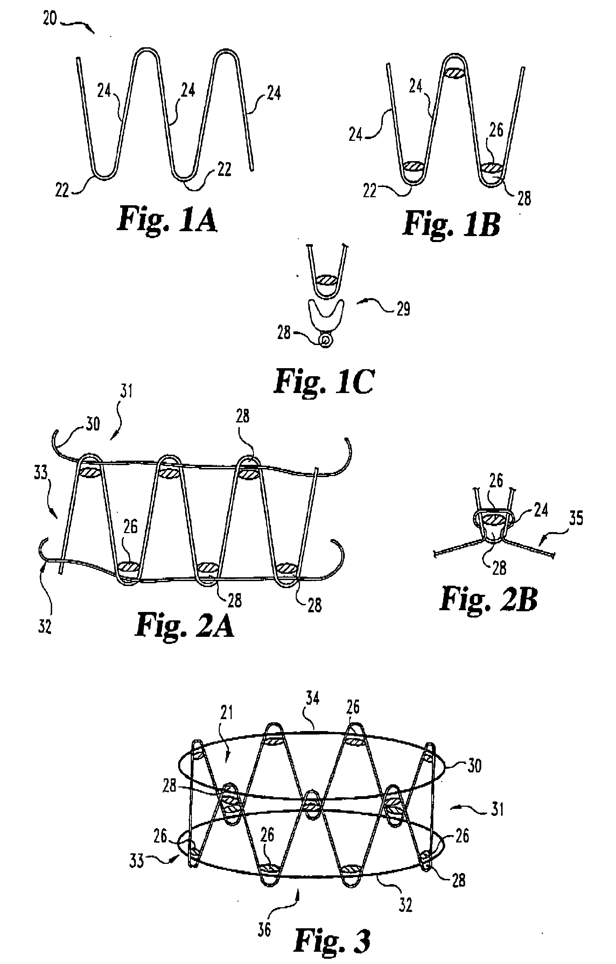

With respect to FIGs. 1A, 1B, and 1C, a frame is partially shown. The frame can comprise a stent 20. Choices of stent include a self expanding stent or a non-self expanding stent. In one embodiment of the present invention stent 20 is a self expanding stent such as the Gianturco stent available from Cook Inc. of Bloomington, IN as described in U.S. patent 4,580,568. Such stent can be any length, but in one embodiment, the stent is about 15 mm long. Stent 20 includes a plurality of bends 22, which generally form the area in which the stent struts 24 reverses direction. Bends 22 are generally rounded to provide an traumatic condition. Since the stent 20 is generally located in a vessel or body lumen of some type, the stent 20 is cylindrical and therefore has a stent diameter 21 (shown in FIG. 3). In another embodiment, the stent 20 can also have a plurality of connectors 26 that connect adjacent struts 24. One way to provide a connector 26 is to dispose a solder bead between the adjacent struts. However connector 26 can also be a suture, weld, adhesive, rod, clamp, or other well-known ways to connect adjacent struts 24. Connector 26 provides several non-critical advantages.

Connectors 26 can attach adjacent struts 24 to minimize or prevent flaring of the ends of the stent 20. Furthermore, connector 26, if placed near the bend 22, can create a hole 28 wherein the boundaries of the hole are the wires of the stent operating in general conjunction with the connector 26. This creates a hole 28 through which a thread or suture can run. However, as shown in FIG. 1C, a separate prefabricated hole can be created by separately attaching a hole assembly, such as a cap 29 over the bend 22. In any case, one benefit of the connector 26 or cap 29 is that they increase the radiographic visualization of the invention. Particularly, if the connector 26 is a solder bead, it has increased radiopacity.

With respect to FIGs. 2A and 2B, shown is part of the stent in which connector 26 attaches adjacent struts 24. As mentioned above, a thread or suture can be threaded through the hole 28. A proximal suture 30 can be sewn through the stent proximal bends 22 or stent proximal ends 31 of the stent. Similarly, a distal suture 32 can be sewn through the stent distal end 33 or the stent distal bends 22 of the stent. One way to thread the suture is shown in FIG. 2B wherein the suture 35 (generically any suture) runs over the strut 24 to enter the hole 28, through hole 28 to come behind the same strut 24, over the strut 24 and across to the adjacent strut 24 running over the adjacent strut 24, behind the adjacent strut 24 to come from behind and through hole 28, and then run subsequently over adjacent strut 24. Once the struts are connected via the suture, the suture can be pulled to a predetermined tightness to control the overall stent size. Accordingly, the stent can be so constructed to have a predetermined stent perimeter 34. To this end, the stent lumen 36 will also have an appropriate size. The stent can be constructed so as to have a different perimeter length at the proximal or distal ends.

With regard to FIG. 3, shown is a cylindrical stent 20 that has the proximal and distal sutures running through the bends 22 or holes 28 of the proximal and distal ends of the stent. By altering the tautness of the sutures, the size of the stent lumen 36, the stent diameter 86, and the stent perimeters 34, can be adjusted. As can be seen, distal perimeter suture 32 runs along the stent distal end 33, whereas proximal perimeter suture 30 runs along the stent proximal end 31. The respective sutures run through hole 28 of each bend 22.

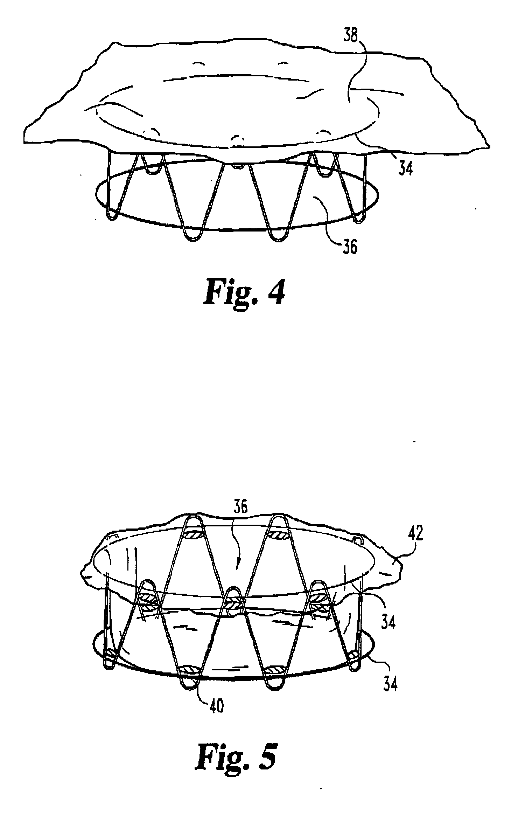

With respect to FIGs. 4 and 5, the valve material 38 is shown, in this exemplary embodiment, as a sheet. In so constructing the valve 41, the valve material 38 is draped across the stent lumen 36 opening (such as shown on the proximal portion of the stent) and then pushed down into the stent lumen 36 itself. Excess material can be kept outside the stent, which will later become a potential fold-over 42. However, the excess material can also be trimmed off. The valve material 38 is connected to the stent, using for example, distal valve-stent suture 40. However, any well known ways to connect the valve to the stent is contemplated, such as but not limited to, sutures, adhesives, folds, or the like. In one embodiment shown in FIG. 5, the valve-stent suture 40 can share the hole 28 with distal suture 32 near the stent perimeter 34.

The valve material 38 can be any biocompatible material such as polyethylene terephalate (PET), polypropylene (PP), polytetrafluorethylene (PTFE), or any polymer or derivative thereof, and also includes commercially known materials such as GORE-TEX, DACRON, or any other synthetic material. The preferred material 38 will be advantageously compliant and employed so as to permit effective value function as described herein and in the case of collapsible/expandable state devices will retain integrity and function when cycled between tehse states.

It is preferred to use a biomaterial that serves as a biocompatible scaffold with the ability to remodel host tissue. Accordingly, a naturally occurring biomaterial is highly desirable. One such biomaterial is collagen and more particularly, a collagen based biomaterial called extracellular matrix (ECM). Examples of ECM's include pericardium, stomach submucosa, liver basement membrane, urinary bladder submucosa, tissue mucosa, dura mater, and small intestine submucosa One such biomaterial is the ECM, such as submucosa, and more particularly is small intestine submucosa (SIS). SIS can be made in the fashion described in Badylak et al., US Patent 4,902,508; Intestinal Collagen Layer described in US Patent 5,733,337 to Carr and in 17 Nature Biotechnology 1083 (Nov. 1999); Cook et al., WIPO Publication WO 98/22158, dated 28 May 1998, which is the published application of PCT/US97/14855; Gastric Submucosa as described in WO 98/26291 (PCT/US97/22729), claiming priority to US Provisional application 60/032,686; Liver tissue as described in WO 98/25637 (PCT/US97/22727), claiming priority to 60/032,680; Stomach Submucosa as described in WO 98/25636 (PCT/US97/23010), claiming priority to 60/032,683; and Urinary Bladder Submucosa as described in US Patent 5,554,389; Irrespective of the origin of the valve material (synthetic versus naturally occurring), the valve material can be made thicker by making multilaminate constructs, for example SIS constructs as described in US Patents 5,968,096; 5,955,110; 5,885,619; and 5,711,969.

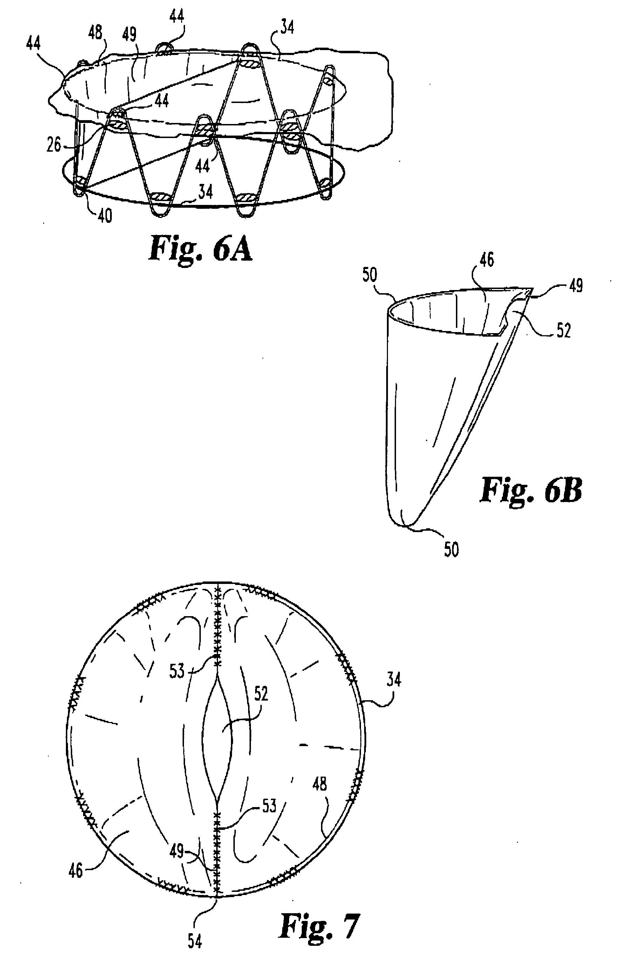

With respect to FIGs. 6A and 6B, shown is the connection of the valve to the stent frame. As described above, the valve can be sutured at the distal portion of the stent using distal valve-stent suture 40. Similarly, the proximal portion of the valve can be sutured to proximal portion of the stent, and more particularly to proximal perimeter suture 30. Shown is the valve connected to the proximal portion of the stent at proximal valve-stent suture 44. Suture 44 can be through a bend 22 or can attach to the proximal perimeter suture 30. In a traditional Gianturco Z-stent, it is either an 8 (bend) point or 10 (bend) point stent, so one leaflet of the valve can be sutured to the four points of an 8 point stent thereby comprising one half of the stent. To provide further integrity, the valve can be sutured at the proximal and distal end to the perimeter sutures themselves, without actually being sutured to any or all of the stent bends 22.

With respect to FIG. 6B, shown is the valve with the stent frame removed. Once the sutures are generally in place, the valve sheet 38 will form a valve pocket 46, extending inside the stent lumen in which the fluid will fill. Proximal valve perimeter 48 will have the sutures connecting the valve to the stent (not shown). Once the distal sutures are in place, the general shape will likely resemble a pocket with the pocket having a valve apex 50. There is a part of the valve that will form central valve portion 49 that is not directly sutured to the stent. This valve portion 49 will form the valve opening 52 through which fluid will pass. Thus, upon filling of the valve pocket 46, the fluid pressure will exert outwards causing valve portion 49 to extend outward. When it does, it will contact the other leaflets or cusps and form a seal to stop or impede fluid flow.

FIG. 7 shows a top view of the stent valve. In this particular non-limiting view, shown is the valve opening 52 in a slightly open configuration. Valve pockets 46 are shown in a slightly distended configuration. The valve is connected, for example, by sutures to the stent perimeter 34 and also forms a valve perimeter 48. Because of the opening and closing of the valve, there may be increased wear and tear at the valve-stent-opening connection. At this point, the present invention provides a reinforcement at this point. For example, this reinforcement can be a plurality of reinforcement sutures 54, adhesive, another material, or any other mechanism that permits increased structural integrity.

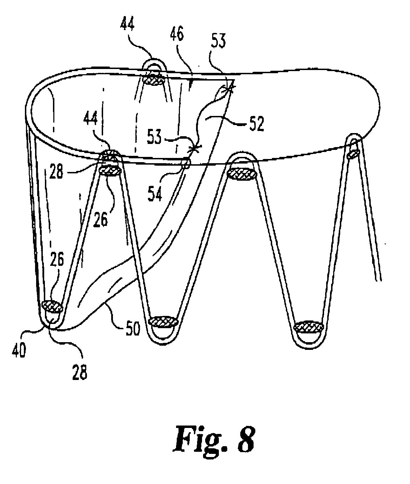

FIG. 8 demonstrates a view of the stent valve once the distal portion of the valve is sewn to a distal bend 22 and also shows the proximal portion of the valve being connected to the proximal portion of the frame with one suture in the foreground, one suture in the background. In addition, the reinforcement suture 54 is found in the foreground. Although only two sutures 44 are seen at the proximal portion, it is of course well-understood that some or each of the proximal bend of the frame can be connected to the proximal portion of the valve. Similarly, although only one distal suture 40 is shown, there may be as many distal sutures necessary to connect the valve apex 50 or the distal portion of the valve to the frame. It is well understood that this may be just one distal suture or many distal sutures. Varying the number of distal sutures will vary the shape, tightness, and overall configuration of the valve, valve pocket 46, and the valve apex 50.

The valve opening 52 although already described above, is actually created in the final step of preparation of the preferred device manufacture. The construction mentioned above would be repeated on the other side of the valve to create the valve pocket 46, valve apex 50, and the like on the other side. At this point, though, there is no valve opening 52. The valve opening 52 is created by creating a slit in the sheet to create the opening. The slit can be sized according to the intended flow rate of the passing fluid. Accordingly, a large slit would create a large valve opening or orifice and permits a large volume of fluid to pass therethrough. The slit can be created by poking a scalpel through it and running it to the desired length. However, due to potential fatigue at the orifice, another set of reinforcements may be added to the orifice perimeter. Therefore, as shown in FIGs. 7 and 8, an orifice reinforcement 53 may be created by any known conventional ways, such as sutures (resorbable or non-resorbable), adhesive, string, staples, rings, or the like.

Therefore, the stent valve as constructed can be using one stent with the valve material enclosed therein. Of course in the single stent configuration, the overall length can be adjusted by elongating the length of the struts 24. However, devices of the invention can be built using a plurality of stents to elongate the overall size of the stent, if desired. In this regard, it will be preferred that the length of the device 20 is sufficient to provide an aspect ratio (length to expanded diameter) sufficiently high to facilitate proper alignment of the device 20 within the vessel, with the axis of the device lumen generally aligned with the axis of the vessel. For example, devices having a ratio of length:expanded diameter of 1:1 or greater, or about 2:1 or greater, will be preferred. It will be understood that while such dimensions will advantageously facilitate placement of the inventive devices, they are not necessary to the broader aspects of the invention.

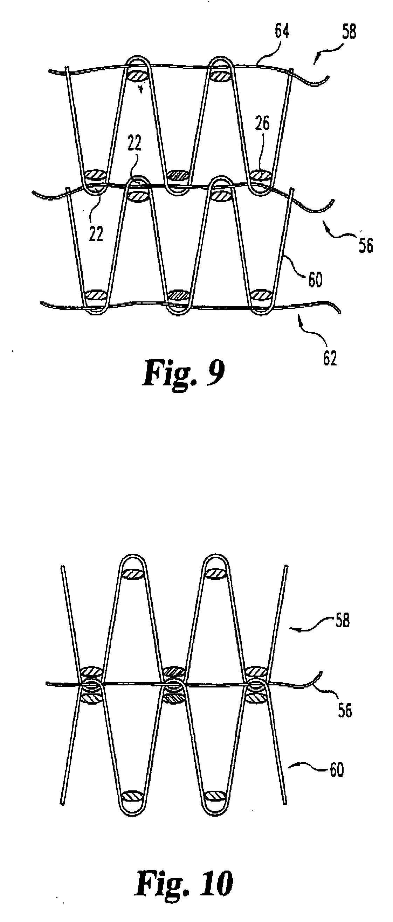

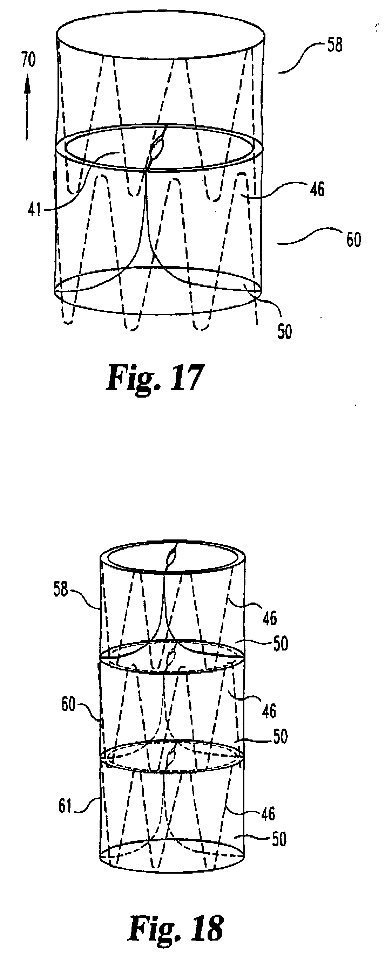

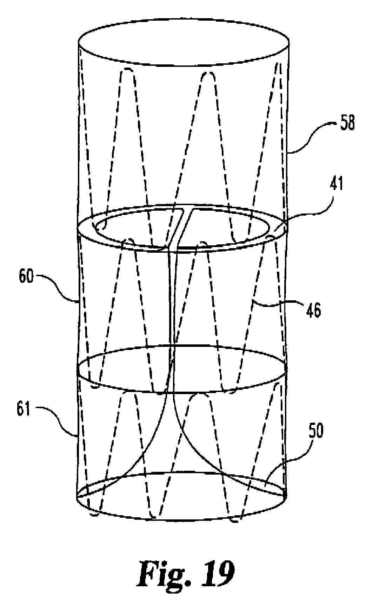

With reference to FIG. 15, shown is a double stent structure with the valve. Returning now to FIG. 9, shown is a first stent 58 and a second stent 60. For the purposes of discussion only, first stent 58 is shown to be atop of the second stent 60. Ultimately as shown herein by way of example only, the valve will reside in the first stent 58. It should be noted however that the valve can reside in the second stent 60 also as shown in FIG. 17. Furthermore, the overall length can be increased by joining several stent valves together as shown in FIGs. 18 and 19, thereby having a plurality of stents, such as a first stent 58, second stent 60, and a third stent 61. The valve 41 can be placed in any or all stents, in any combination, for example, as shown by the dotted lines. In this regard, it is suggested and intended that many stents can be joined and that each or any stent may house a valve or plurality of valves. One benefit of having a plurality of stents is that upon ejection of the placement device, the invention will provide a self-aligning feature in the vessel. This is because the plurality of stents is generally longer with respect to the stent diameter, or the plurality of valve device(s), as discussed above.

Manufacture of the multi-stent or multi-valve device will generally follow the same construction as described above. The same considerations in making a single valve single stent device applies equally to the elongated device.

Returning now to FIGs. 9 and 10, shown are methods of connecting the first stent 58 and second stent 60. Equally, the construction shown from now on also includes construction of at least two stents or at least two valves. First stent 58 and second stent 60 has bends 22 that are adjacent each other. Shown in FIG. 9 is where the first stent 58 has its bends beside the bends of the second stent 60 such that they are not touching each other (although they may touch). They are connected together in the manner described above, and for example by stent-stent suture 56. Stent-stent suture 56 can be resorbable or non-resorbable. This suture travels through the distal bends of the first stent 56 and the proximal bends of the second stent 60. The suturing pattern can be that described in FIG. 2B and the accompanying discussion. As shown in FIG. 10, the bends can be juxtaposed over each other to provide an overlap such that the stent-stent suture 56 will go through the bends at the same time. Therefore, the construction contemplates that the stent bends may touch, overlap, or not at all.

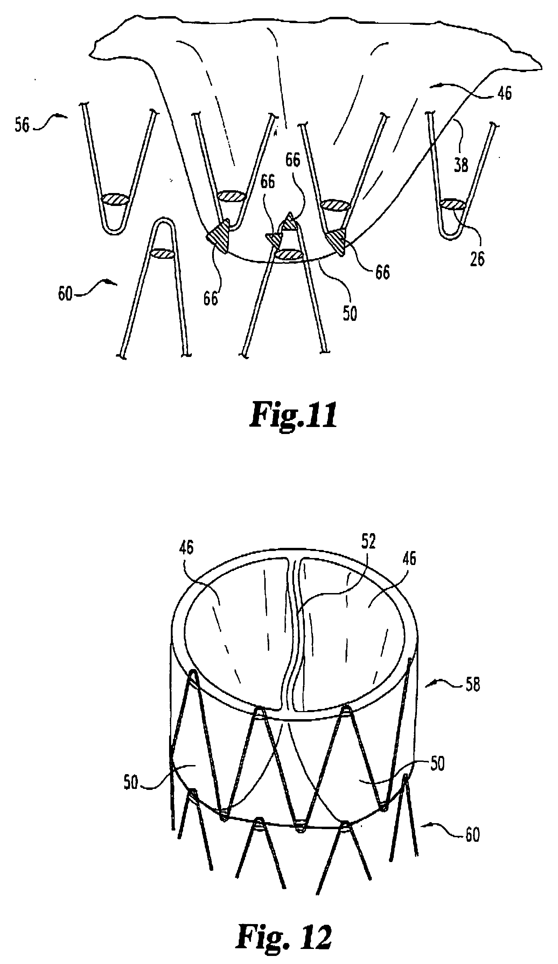

FIG. 11 shows one embodiment of the present invention in which the valve apex 50 is sutured to at least three bends: two bends of the first stent 56 and one bend of the second stent 60. In this regard, the valve also operates to keep the first stent 56 partially connected to the second stent 60. From the bends, a plurality of valve apex sutures 66 are seen. These sutures can emanate from the bends and each bend can have many valve apex sutures 66 that travel in many directions. Using multiple valve apex sutures 66 that emanate in many directions and using a plurality of bends (from either stent), generally functions to minimize any parachuting or inversion of the valve pocket 46.

FIG. 12 demonstrates a top view of the multi-stent device in which the valve opening 52 is seen (in a closed position) and the valve pocket 46 and valve apex 50 is connected to three bends. Again it should be understood that many sutures may emanate from many bends from any stent.

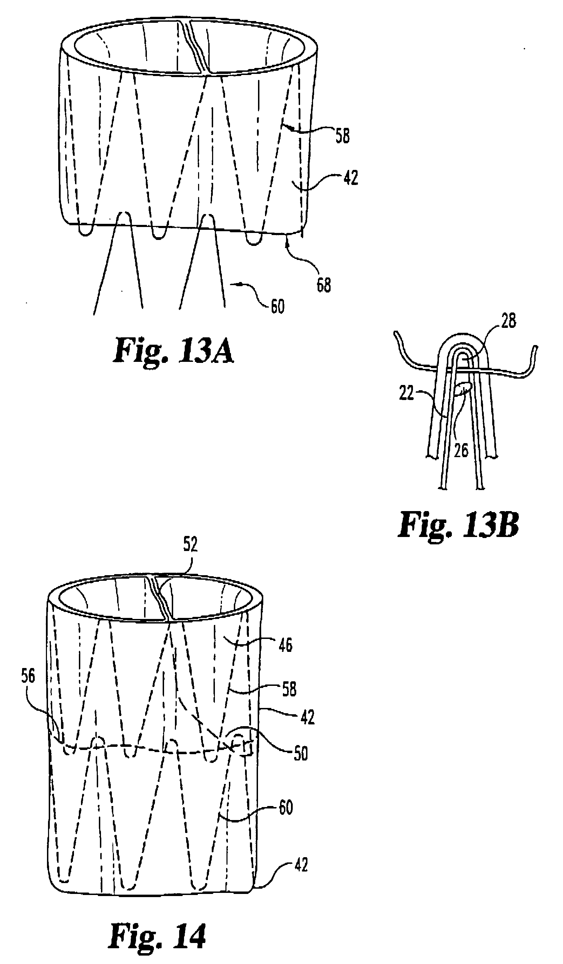

As described earlier, the excess material can either be trimmed off or folded over the outer surface of the device. Shown in FIGs. 13A and 13B, is the excess material being folded over the device and attached at the distal end of the first stent 58. Shown in dotted lines is the first stent 58. FIG. 13B shows that the fold-over 42 provides a second material outer sheath so that the suture passes through the inside and outside material to increase structural integrity. Also, by folding over the excess material, a smoother surface is presented rather than the naked frame of the tip of the bend.

In all embodiments of the invention, the external surface of the frame can be covered with a sheath that is not necessarily the same material as the valve 41. For example, while the valve can be a naturally occurring material, the outer sheath can be synthetic material such as described herein. The sheath, therefore, can be the fold-over of the valve material, another type of naturally occurring material, or a synthetic material. Accordingly, the sheath may partially or totally cover the frame.

FIG. 14 shows an embodiment in which both the first stent 58 and second stent 60 are covered by the fold over 42. Here, the fold-over 42 is connected to the distal portion of the second stent 60. In this manner, the entire device may be covered with an outer sheath of biomaterial. The benefit of doing so, especially if using SIS or other similar ECMs, is that the regrowth and endothelialization of the device embeds and encapsulates the frame. Accordingly, there is a reduced risk of device migration. Furthermore, due to the remarkable remodeling properties of SIS, the outer SIS sheath acts as a conduit for host tissue to infiltrate the device and remodel the valve itself. Over the course of months, the valves are replaced by host tissue and the SIS disappears.

FIG. 15 shows yet another embodiment of the present invention. In this demonstration, the valve is located in the first stent 58, sutured at the proximal end at the stent perimeter. The valve apex 50 is sewn somewhat proximal of the stent-stent suture 56. The valve apex 50 is sewn at the valve apex sutures 66 to an intermediate portion of the frame. To minimize parachuting or inversion, a valve intermediate portion 75 may be sutured using valve intermediate suture 76 to connect the valve to the frame. In addition, the valve may be so constructed to extend the valve's length to create an elongated valve pocket 90 (shown by the dotted lines). While the extended pocket 90 can be connected to the distal perimeter of the second stent distal, suture 62, it can also be connected to an intermediate portion of the second stent.

With further reference to FIG. 15, it is seen that the valve opening 52 is a slit that extends across the first stent diameter 21 but terminates several millimeters before reaching the edge. In some embodiments, this distance could be 1-5 mm from the edge. Of course, it is understood that the invention contemplates any distance that varies the length of the slit. Also, shown in FIG. 15, but equally applies to any device described herein, is an anchor 92, which can be anchor barbs 92. These barbs 92 can dig into the adjacent vessel wall to relatively affix the device at its location. Anchor 92, although shown as barbs, may include hooks, adhesives, knobs, a textured surface, or any other treated surface that facilitates relative affixation of the device in its location. Similarly, the outer surface of the fold-over or sheath can be so configured to provide anchoring.

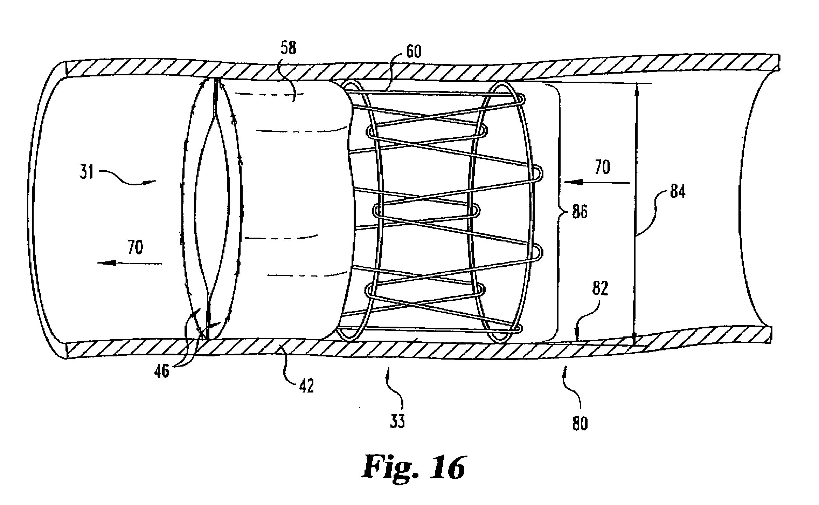

FIG. 16 demonstrates the device upon implantation into the patient. Upon implantation the device generally resides in a vessel 80. For example, the vessel could be vein, artery or the heart or wherever a valve is necessary. In one preferred use, the vessel is an incompetent vein in the leg or foot of a patient. The device 20 reduces or prevents retrograde blood flow, while normal blood flow is permitted to travel through device 20. Illustrative veins in which the device 20 may be used include, for example, saphenous veins, femoral veins, popliteal veins, tibial veins, and the inferior vena cava.

The vessel 80 has an inner lumenal surface 82 in which the fluid flows. The fluid flow path is shown as fluid path 70. Vessel 80 also has a vessel diameter 84. The medical device, upon implantation, will also have a device outer stent diameter 86. The outer diameter 86 will be chosen to permit contact with the inner lumenal surface 82. The optimized fit will decrease the leakage around the device by contacting the inner lumenal surface 82. A tight fit can be accomplished by sizing the stent diameter to be greater than the vessel diameter. For example, a stent diameter that is about 110 percent greater than (i.e. 1.1 times) the vessel diameter provides a good fit. Expanded stent diameters of about 10 mm to about 30 mm will be typical in many applications of the present invention.

Again, while it is shown in this FIG. 16 that the valve is located in the first stent 58 and only the first stent 58 is covered by the fold-over 42 or sheath, it should be remembered that the valve could be located in the second stent 60. Similarly, the fold-over 42 or sheath could extend onto the second stent 60.

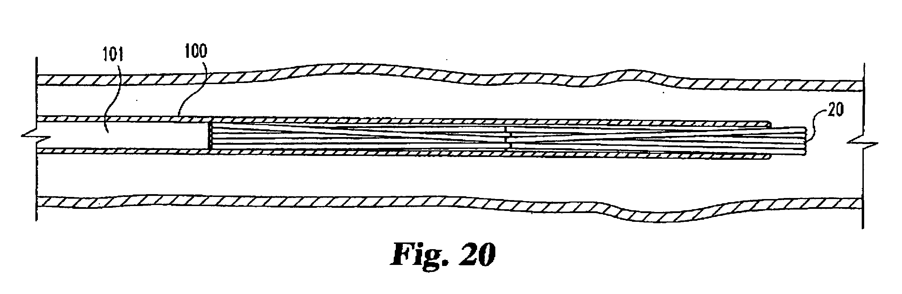

The standard method of deploying the medical device 20 in a vessel 80 involves the use of a medical assembly (see FIG. 20) including the device 20 and a delivery device such as a percutaneous delivery device, e.g. a catheter 100. The frame is configured to a contracted state, e.g. by resiliently forming the frame into a contracted configuration, to load into the delivery device (catheter). The catheter can be introduced into the patient via a suitable approach, for example through the jugular or femoral vein. To advance and deploy the device from the distal end of the delivery catheter, a pusher 101 is placed into the catheter lumen. When the device 20 is fully deployed, it assumes the second, expanded configuration within the vessel 80 as depicted in FIG. 16. The stent frame, being made of resilient material, conforms to the shape of the vessel wall such that when viewed on end, the device 20 has a circular appearance when deployed in a round vessel.

FIGs. 17, 18, and 19 show other described embodiments. FIG. 17 demonstrates the valve 41 in the second stent 60. In this embodiment, the valve apex 50 is connected to the second stent's distal perimeter. FIG. 18 demonstrates at least two stent frames connected together. In this particular embodiment, the valve is located in the first stent 58, with the valve apex 50 being connected at the first stent 58-second stent 60 junction. In dotted lines, however, there may be many stents, such as first stent 58, second stent 60, and third stent 61. The valve 41 may be found in any of the stents or in all. Similarly, in the three stent configuration, the valve may begin at the first stent and have the valve apex 50 be generally located in the third stent 61. FIG. 19 shows another embodiment of the present invention in which the valve 41 begins in the second stent 60 and extends into the third stent 61 thereby having the first stent 58 being empty.

Finally, since the device is to be located in an *in vivo* environment, the device may be treated with therapeutic agents to facilitate healing. For example, the frame may be treated with therapeutic agents such as anti-cancer drugs, plaque busters, anti-coagulants, or the like. Similarly, the valve material can be treated with therapeutics agents such as anti-cancer drugs, plaque busters, anti-coagulants, proteins, growth factors, proteoglycans, and the like. Furthermore, radiopaque agents may be added, such as tantalum, barium, bismuth, or the like to increase radiopacity. These ingredients can be bonded to the frame or the valve material such as rubbing the agent in, bonding it, adhering it, or the like.

While the invention has been illustrated and described in detail in the drawings and the foregoing text, it is understood that these are only some embodiments and that the scope of the invention is not solely defined by the description herein but also by the appended claims.

## Claims

1. A stent valve device for delivery by catheter (100) to a vessel site (80) in the body, comprising:
a radially expandable cylindrical stent (20), the radially expandable cylindrical stent having a collapsed configuration for delivery and an expanded configuration for deployment at the vessel site (80), the radially expandable cylindrical stent (20) having a proximal stent end (31), a distal stent end (33), a stent lumen (36), and a stent diameter (21) and a stent perimeter (34),
a valve (41) located within the lumen (36) of the radially expandable cylindrical stent (20), the valve (41) comprising biocompatible material (38) and at least two leaflets, the valve (41) having a valve orifice (52) between the at least two leaflets, the valve orifice (52) extending substantially across the stent diameter (21), wherein
(a) the valve (41) has a proximal portion connected to a proximal portion (31) of the radially expandable cylindrical stent (20) and a distal portion connected to a distal portion (33) of the radially expandable cylindrical stent (20);
(b) at the locations between adjacent leaflets, the valve orifice (52) terminates 1 to 5 millimeters before reaching the perimeter (34) of the radially expandable cylindrical stent (20);
(c) the valve material (38) is enclosed within the radially expandable cylindrical stent (20);
**characterized in that**
(d) reinforcement mechanisms (54) are located between the orifice perimeter and the stent (20) perimeter at the locations between adjacent leaflets at valve-stent-opening connection points; thereby reinforcing said connection points against wear and tear due to opening and closing of the valve and permitting increased structural integrity of the stent valve device.

2. The stent valve device of claim 1, further comprising a sheath which covers the stent (20).

3. The stent valve device of claim 1, further comprising a sheath which partially covers the stent.

4. The stent valve device of claim 2 or claim 3, wherein the sheath comprises a material that is not the same as the valve material (38).

5. The stent device of claim 4, wherein the valve (41) is comprised of a naturally occurring material, and the sheath is comprised of a synthetic material.

6. The stent valve device of claim 2 or 3, wherein the sheath comprises a synthetic material.

7. The stent valve device of claim 5 or 6, wherein the synthetic material is polyethylene terephthalate.

8. The stent valve device of any preceding claim, wherein the valve comprises two, three or four leaflets.

9. The stent valve device of any one of claims 1 to 8 wherein the valve material comprises pericardium.

10. The stent valve device of any one of claims 1 to 7, wherein the leaflets comprise pericardium.

11. The stent valve device of any one of claims 1 to 10, wherein the radially expandable cylindrical stent (20) is approximately 15 mm long.

12. The stent valve device of any one of claims 1 to 11, wherein the radially expandable cylindrical stent (20) is a non-self-expanding stent.

13. The stent valve device of any one of claim 1 to 11, wherein the stent (20) is a self-expanding stent.

14. The stent valve device of any one of claims 1 to 13, wherein the stent valve device has only a single radially expandable cylindrical stent (20).

15. The stent valve device of claim 2 or 3, wherein the outer surface of the sheath is configured to provide anchoring of the device.

16. The stent valve device of any one of claims 1 to 15 wherein said distal portion of the valve (41) is sutured to said distal portion (33) of the stent (20).

17. The stent valve device of any one of claims 1 to 16, wherein said proximal portion of the valve (41) is sutured to said proximal portion (31) of the stent (20).

18. The stent valve device of claim 15, wherein the outer surface of the sheath provides a textured surface.

19. The stent valve device of claim 18, wherein the reinforcement mechanisms comprise sutures, adhesive, or another material.

20. The stent valve device of claim 19, wherein the reinforcement mechanisms (54) comprise sutures.

21. The stent valve device of claim 19, wherein the reinforcement mechanisms (54) comprise adhesive reinforcements.

22. The stent valve device of claim 19, wherein the reinforcement mechanisms (54) comprise a material other than sutures or adhesive.

23. The stent valve device of any of claims 18 to 22, wherein the reinforcement mechanisms reinforce upper portions of adjacent leaflets in contact with one another.

24. The sent valve device of any one of claims 1 to 17, also comprising a reinforcement (53) disposed on the valve orifice (52).

25. The stent valve device of claim 24, wherein the reinforcement (53) comprises sutures, adhesive, string, staples or rings.

26. The stent valve device of any of claims 1 to 3 wherein the valve (41) comprises polyethylene terephthalate.

27. The stent valve device of any one of claims 1 to 26, wherein the valve material (38) is made thicker by making multilaminate constructs.

28. The stent valve device of any one of claims 1 to 27, wherein the stent (20) is treated with a therapeutic agent.

29. The stent valve device of claim 28, wherein the therapeutic agent is an anticoagulant.

30. The stent valve device of any one of claims 1 to 29, wherein the valve (41) is treated with a therapeutic agent.

31. The stent valve device of claim 30, wherein the therapeutic agent with which the valve is treated is an anticoagulant.

32. The stent valve device of any one of claims 1 to 31, for delivery by catheter (100) to a vessel site (80) in a vein, artery or heart.

33. The stent valve device of any of claims 1 to 31, for delivery by catheter (100) to a vessel site (80) in a heart.

34. The stent valve device of any of claims 1 to 31, for delivery by catheter (100) to a vessel site (80) in a vein.

35. The stent valve device of any of claims 1 to 31, for delivery by catheter (100) to a vessel site (80) in an artery.

36. The stent valve device of any preceding claim, in combination with a catheter (100) for delivery of the device.

37. The stent valve device of claim 36 received in a contracted configuration in the catheter (100).

## Patentansprüche

1. Stentklappenvorrichtung zur Zuführung durch einen Katheter (100) zu einer Gefäßstelle (80) in dem Körper, wobei die Vorrichtung Folgendes umfasst:
einen radial ausdehnbaren zylindrischen Stent (20), wobei der radial ausdehnbare zylindrische Stent eine zusammengefallene Konfiguration zur Zuführung und eine ausgedehnte Konfiguration zur Entfaltung an der Gefäßstelle (80) hat, wobei der radial ausdehnbare zylindrische Stent (20) ein proximales Stentende (31), ein distales Stentende (33), ein Stentlumen (36) und einen Stentdurchmesser (21) und einen Stentumfang (34) hat,
eine Klappe (41), die innerhalb des Lumens (36) des radial ausdehnbaren zylindrischen Stents (20) angeordnet ist, wobei die Klappe (41) biologisch verträgliches Material (38) und wenigstens zwei Segel umfasst, wobei die Klappe (41) eine Klappenöffnung (52) zwischen den wenigstens zwei Segeln hat, wobei sich die Klappenöffnung (52) im Wesentlichen über den Stentdurchmesser (21) erstreckt, wobei
(a) die Klappe (41) einen proximalen Abschnitt, der mit einem proximalen Abschnitt (31) des radial ausdehnbaren zylindrischen Stents (20) verbunden ist, und einen distalen Abschnitt, der mit einem distalen Abschnitt (33) des radial ausdehnbaren zylindrischen Stents (20) verbunden ist, hat,
(b) an den Positionen zwischen benachbarten Segeln die Klappenöffnung (52) 1 bis 5 Millimeter vor dem Erreichen des Umfangs (34) des radial ausdehnbaren zylindrischen Stents (20) endet,
(c) das Klappenmaterial (38) innerhalb des radial ausdehnbaren zylindrischen Stents (20) eingeschlossen ist,
**dadurch gekennzeichnet, dass**
(d) Verstärkungsmechanismen (54) zwischen dem Öffnungsumfang und dem Umfang des Stents (20), an den Positionen zwischen benachbarten Segeln an Klappen-Stent-Öffnung-Verbindungspunkten angeordnet sind, wodurch die Verbindungspunkte gegen Verschleiß und Abnutzung auf Grund des Öffnens und Schließens der Klappe verstärkt werden und eine gesteigerte strukturelle Integrität der Stentklappenvorrichtung ermöglicht wird.

2. Stentklappenvorrichtung nach Anspruch 1, die ferner eine Hülse umfasst, die den Stent (20) abdeckt.

3. Stentklappenvorrichtung nach Anspruch 1, die ferner eine Hülse umfasst, die den Stent teilweise abdeckt.

4. Stentklappenvorrichtung nach Anspruch 2 oder Anspruch 3, wobei die Hülse ein Material umfasst, das nicht das gleiche ist wie das Klappenmaterial (38).

5. Stentklappenvorrichtung nach Anspruch 4, wobei die Klappe (41) aus einem natürlich vorkommenden Material besteht und die Hülse aus einem synthetischen Material besteht.

6. Stentklappenvorrichtung nach Anspruch 2 oder 3, wobei die Hülse ein synthetisches Material umfasst.

7. Stentklappenvorrichtung nach Anspruch 5 oder 6, wobei das synthetische Material Polyethylenterephthalat ist.

8. Stentklappenvorrichtung nach einem der vorhergehenden Ansprüche, wobei die Klappe zwei, drei oder vier Segel umfasst.

9. Stentklappenvorrichtung nach einem der Ansprüche 1 bis 8, wobei das Klappenmaterial Perikard umfasst.

10. Stentklappenvorrichtung nach einem der Ansprüche 1 bis 7, wobei die Segel Perikard umfassen.

11. Stentklappenvorrichtung nach einem der Ansprüche 1 bis 10, wobei der radial ausdehnbare zylindrische Stent (20) ungefähr 15 mm lang ist.

12. Stentklappenvorrichtung nach einem der Ansprüche 1 bis 11, wobei der radial ausdehnbare zylindrische Stent (20) ein nicht-selbstausdehnender Stent ist.

13. Stentklappenvorrichtung nach einem der Ansprüche 1 bis 11, wobei der Stent (20) ein selbstausdehnender Stent ist.

14. Stentklappenvorrichtung nach einem der Ansprüche 1 bis 13, wobei die Stentklappenvorrichtung nur einen einzigen radial ausdehnbaren zylindrischen Stent (20) hat.

15. Stentklappenvorrichtung nach Anspruch 2 oder 3, wobei die Außenfläche der Hülse dafür konfiguriert ist, eine Verankerung der Vorrichtung zu gewährleisten.

16. Stentklappenvorrichtung nach einem der Ansprüche 1 bis 15, wobei der distale Abschnitt der Klappe (41) an den distalen Abschnitt (33) des Stents (20) genäht ist.

17. Stentklappenvorrichtung nach einem der Ansprüche 1 bis 16, wobei der proximale Abschnitt der Klappe (41) an den proximalen Abschnitt (31) des Stents (20) genäht ist.

18. Stentklappenvorrichtung nach Anspruch 15, wobei die Außenfläche der Hülse eine texturierte Oberfläche bereitstellt.

19. Stentklappenvorrichtung nach Anspruch 18, wobei die Verstärkungsmechanismen Nähte, Klebstoff oder ein anderes Material umfassen.

20. Stentklappenvorrichtung nach Anspruch 19, wobei die Verstärkungsmechanismen (54) Nähte umfassen.

21. Stentklappenvorrichtung nach Anspruch 19, wobei die Verstärkungsmechanismen (54) Klebstoffverstärkungen umfassen.

22. Stentklappenvorrichtung nach Anspruch 19, wobei die Verstärkungsmechanismen (54) ein anderes Material als Nähte oder Klebstoff umfassen.

23. Stentklappenvorrichtung nach einem der Ansprüche 18 bis 22, wobei die Verstärkungsmechanismen die oberen Abschnitte der benachbarten Segel in Berührung miteinander verstärken.

24. Stentklappenvorrichtung nach einem der Ansprüche 1 bis 17, die ebenfalls eine Verstärkung (53) umfasst, die an der Klappenöffnung (52) angeordnet ist.

25. Stentklappenvorrichtung nach Anspruch 24, wobei die Verstärkung (53) Nähte, Klebstoff, Schnur, Klammern oder Ringe umfasst.

26. Stentklappenvorrichtung nach einem der Ansprüche 1 bis 3, wobei die Klappe (41) Polyethylenterephthalat umfasst.

27. Stentklappenvorrichtung nach einem der Ansprüche 1 bis 26, wobei das Klappenmaterial (38) durch das Herstellen von Multilaminataufbauten dicker hergestellt ist.

28. Stentklappenvorrichtung nach einem der Ansprüche 1 bis 27, wobei der Stent (20) mit einem therapeutischen Wirkstoff behandelt ist.

29. Stentklappenvorrichtung nach Anspruch 28, wobei der therapeutische Wirkstoff ein Gerinnungshemmer ist.

30. Stentklappenvorrichtung nach einem der Ansprüche 1 bis 29, wobei die Klappe (41) mit einem therapeutischen Wirkstoff behandelt ist.

31. Stentklappenvorrichtung nach Anspruch 30, wobei der therapeutische Wirkstoff, mit dem die Klappe behandelt ist, ein Gerinnungshemmer ist.

32. Stentklappenvorrichtung nach einem der Ansprüche 1 bis 31, zur Zuführung durch einen Katheter (100) zu einer Gefäßstelle (80) in einer Vene, einer Arterie oder einem Herzen.

33. Stentklappenvorrichtung nach einem der Ansprüche 1 bis 31, zur Zuführung durch einen Katheter (100) zu einer Gefäßstelle (80) in einem Herzen.

34. Stentklappenvorrichtung nach einem der Ansprüche 1 bis 31, zur Zuführung durch einen Katheter (100) zu einer Gefäßstelle (80) in einer Vene.

35. Stentklappenvorrichtung nach einem der Ansprüche 1 bis 31, zur Zuführung durch einen Katheter (100) zu einer Gefäßstelle (80) in einer Arterie.

36. Stentklappenvorrichtung nach einem der vorhergehenden Ansprüche, in Kombination mit einem Katheter (100) zur Zuführung der Vorrichtung.

37. Stentklappenvorrichtung nach Anspruch 36, die in einer kontrahierten Konfiguration in dem Katheter (100) aufgenommen wird.

## Revendications

1. Dispositif de valvule d'endoprothèse vasculaire destiné à être mis en place par cathéter (100) sur un site vasculaire (80) du corps, comprenant :
une endoprothèse vasculaire cylindrique à dilatation radiale (20), l'endoprothèse vasculaire cylindrique à dilatation radiale présentant une configuration repliée pour la mise en place et une configuration dilatée pour le déploiement sur le site vasculaire (80), l'endoprothèse vasculaire cylindrique à dilatation radiale (20) présentant une extrémité proximale d'endoprothèse vasculaire (31), une extrémité distale d'endoprothèse vasculaire (33), une lumière d'endoprothèse vasculaire (36) et un diamètre d'endoprothèse vasculaire (21) et un périmètre d'endoprothèse vasculaire (34),
une valvule (41) située au sein de la lumière (36) de l'endoprothèse vasculaire cylindrique à dilatation radiale (20), la valvule (41) comprenant un matériau biocompatible (38) et au moins deux feuillets, la valvule (41) présentant un orifice de valvule (52) entre les au moins deux feuillets, l'orifice de valvule (52) s'étendant essentiellement à travers le diamètre d'endoprothèse vasculaire (21), dans lequel
(a) la valvule (41) présente une partie proximale connectée à une partie proximale (31) de l'endoprothèse vasculaire cylindrique à dilatation radiale (20) et une partie distale connectée à une partie distale (33) de l'endoprothèse vasculaire cylindrique à dilatation radiale (20) ;
(b) aux emplacements situés entre les feuillets adjacents, l'orifice de valvule (52) se termine entre 1 et 5 millimètres de distance du périmètre (34) de l'endoprothèse vasculaire cylindrique à dilatation radiale (20) ;
(c) le matériau de la valvule (38) est enfermé à l'intérieur de l'endoprothèse vasculaire cylindrique à dilatation radiale (20) ;
**caractérisé en ce que**
(d) des mécanismes de renfort (54) sont situés entre le périmètre de l'orifice et le périmètre de l'endoprothèse vasculaire (20) aux emplacements situés entre les feuillets adjacents en des points de connexion valvule-ouverture d'endoprothèse vasculaire ; en renforçant ainsi lesdits points de connexion contre l'usure due à l'ouverture et à la fermeture de la valvule et en permettant une plus grande intégrité structurelle du dispositif de valvule d'endoprothèse vasculaire.

2. Dispositif de valvule d'endoprothèse vasculaire selon la revendication 1, comprenant en outre une gaine qui recouvre l'endoprothèse vasculaire (20).

3. Dispositif de valvule d'endoprothèse vasculaire selon la revendication 1, comprenant en outre une gaine qui recouvre en partie l'endoprothèse vasculaire.

4. Dispositif de valvule d'endoprothèse vasculaire selon la revendication 2 ou la revendication 3, dans lequel la gaine comprend un matériau qui n'est pas le même que le matériau de la valvule (38).

5. Dispositif de valvule d'endoprothèse vasculaire selon la revendication 4, dans lequel la valvule (41) se compose d'un matériau naturel, et la gaine se compose d'un matériau synthétique.

6. Dispositif de valvule d'endoprothèse vasculaire selon la revendication 2 ou 3, dans lequel la gaine comprend un matériau synthétique.

7. Dispositif de valvule d'endoprothèse vasculaire selon la revendication 5 ou 6, dans lequel le matériau synthétique est le polyéthylène téréphtalate.

8. Dispositif de valvule d'endoprothèse vasculaire selon l'une quelconque des revendications précédentes, dans lequel la valvule comprend deux, trois ou quatre feuillets.

9. Dispositif de valvule d'endoprothèse vasculaire selon l'une quelconque des revendications 1 à 8, dans lequel le matériau de la valvule comprend le péricarde.

10. Dispositif de valvule d'endoprothèse vasculaire selon l'une quelconque des revendications 1 à 7, dans lequel les feuillets comprennent le péricarde.

11. Dispositif de valvule d'endoprothèse vasculaire selon l'une quelconque des revendications 1 à 10, dans lequel l'endoprothèse vasculaire cylindrique à dilatation radiale (20) fait environ 15 mm de long.

12. Dispositif de valvule d'endoprothèse vasculaire selon l'une quelconque des revendications 1 à 11, dans lequel l'endoprothèse vasculaire cylindrique à dilatation radiale (20) est constituée par une endoprothèse vasculaire non dilatable automatiquement.

13. Dispositif de valvule d'endoprothèse vasculaire selon l'une quelconque des revendications 1 à 11, dans lequel l'endoprothèse vasculaire (20) est constituée par une endoprothèse vasculaire à dilatation automatique.

14. Dispositif de valvule d'endoprothèse vasculaire selon l'une quelconque des revendications 1 à 13, dans lequel le dispositif de valvule d'endoprothèse vasculaire ne présente qu'une seule endoprothèse vasculaire cylindrique à dilatation radiale (20).

15. Dispositif de valvule d'endoprothèse vasculaire selon la revendication 2 ou 3, dans lequel la surface extérieure de la gaine est configurée pour permettre un ancrage du dispositif.

16. Dispositif de valvule d'endoprothèse vasculaire selon l'une quelconque des revendications 1 à 15, dans lequel ladite partie distale de la valvule (41) est fixée par suture sur ladite partie distale (33) de l'endoprothèse vasculaire (20).

17. Dispositif de valvule d'endoprothèse vasculaire selon l'une quelconque des revendications 1 à 16, dans lequel ladite partie proximale de la valvule (41) est fixée par suture sur ladite partie proximale (31) de l'endoprothèse vasculaire (20).

18. Dispositif de valvule d'endoprothèse vasculaire selon la revendication 15, dans lequel la surface extérieure de la gaine offre une surface texturée.

19. Dispositif de valvule d'endoprothèse vasculaire selon la revendication 18, dans lequel les mécanismes de renfort comprennent des sutures, des adhésifs ou un autre matériau.

20. Dispositif de valvule d'endoprothèse vasculaire selon la revendication 19, dans lequel les mécanismes de renfort (54) comprennent des sutures.

21. Dispositif de valvule d'endoprothèse vasculaire selon la revendication 19, dans lequel les mécanismes de renfort (54) comprennent des renforcements adhésifs.

22. Dispositif de valvule d'endoprothèse vasculaire selon la revendication 19, dans lequel les mécanismes de renfort (54) comprennent un matériau autre que des sutures ou adhésifs.

23. Dispositif de valvule d'endoprothèse vasculaire selon l'une quelconque des revendications 18 à 22, dans lequel les mécanismes de renfort renforcent des parties supérieures de feuillets adjacents en contact mutuel.

24. Dispositif de valvule d'endoprothèse vasculaire selon l'une quelconque des revendications 1 à 17, comprenant également un renforcement (53) agencé sur l'orifice de valvule (52).

25. Dispositif de valvule d'endoprothèse vasculaire selon la revendication 24, dans lequel le renforcement (53) comprend des sutures, un adhésif, un cordon, des agrafes ou des anneaux.

26. Dispositif de valvule d'endoprothèse vasculaire selon l'une quelconque des revendications 1 à 3, dans lequel la valvule (41) comprend du polyéthylène téréphtalate.

27. Dispositif de valvule d'endoprothèse vasculaire selon l'une quelconque des revendications 1 à 26, dans lequel le matériau de la valvule (38) est rendu plus épais en réalisant une structure multicouche.

28. Dispositif de valvule d'endoprothèse vasculaire selon l'une quelconque des revendications 1 à 27, dans lequel l'endoprothèse vasculaire (20) est traitée par un agent thérapeutique.

29. Dispositif de valvule d'endoprothèse vasculaire selon la revendication 28, dans lequel l'agent thérapeutique est un anticoagulant.

30. Dispositif de valvule d'endoprothèse vasculaire selon l'une quelconque des revendications 1 à 29, dans lequel la valvule (41) est traitée par un agent thérapeutique.

31. Dispositif de valvule d'endoprothèse vasculaire selon la revendication 30, dans lequel l'agent thérapeutique avec lequel la valvule est traitée est un anticoagulant.

32. Dispositif de valvule d'endoprothèse vasculaire selon l'une quelconque des revendications 1 à 31, destiné à être mis en place par cathéter (100) sur un site vasculaire (80) d'une veine, d'une artère ou du coeur.

33. Dispositif de valvule d'endoprothèse vasculaire selon l'une quelconque des revendications 1 à 31, destiné à être mis en place par cathéter (100) sur un site vasculaire (80) du coeur.

34. Dispositif de valvule d'endoprothèse vasculaire selon l'une quelconque des revendications 1 à 31, destiné à être mis en place par cathéter (100) sur un site vasculaire (80) d'une veine.

35. Dispositif de valvule d'endoprothèse vasculaire selon l'une quelconque des revendications 1 à 31, destiné à être mis en place par cathéter (100) sur un site vasculaire (80) d'une artère.

36. Dispositif de valvule d'endoprothèse vasculaire selon l'une quelconque des revendications précédentes, en combinaison avec un cathéter (100) permettant de mettre en place le dispositif.

37. Dispositif de valvule d'endoprothèse vasculaire selon la revendication 36, reçu dans une configuration contractée dans le cathéter (100).
